# EUROPEAN PATENT SPECIFICATION

(11) **EP 1 388 371 B1**
(45) Date of publication and mention of the grant of the patent: **28.10.2009**
(21) Application number: 03077325.3
(22) Date of filing: 22.07.1997
(51) Int. Cl.: B05B 5/025, D01D 5/00, A61F 13/00

(54) **A dispensing device and method for forming material**
Abgabevorrichtung und Verfahren zur Bildung eines Materials
Dispositif de distribution et procédé d'élaboration d'un matériau

(30) Priority: 23.07.1996 GB 9615387; 26.09.1996 GB 9620064
(43) Date of publication of application: 11.02.2004
(62) Divisional of application: 97932918.2
(73) Proprietor: BATTELLE MEMORIAL INSTITUTE, Columbus, OH 43201-2693 (US)
(72) Inventor: Coffee, Ronald Alan, Haslemere Surrey GU27 1HA (GB)
(74) Representative: Clark, Jane Anne

(56) References cited:
- EP-A- 0 005 035
- EP-A- 0 468 736
- WO-A-94/12285
- WO-A-95/26235
- US-A- 4 043 331
- DATABASE WPI Week 9602 Derwent Publications Ltd., London, GB; AN 96-018586 XP002046662 & RU 2 034 534 C (EKOMEDSERVIS), 5 October 1995 (1995-10-05)

## Description

This invention relates to a method of providing a wound dressing for use, for example, in the care or treatment of wounds or burns.

Various forms of aerosol devices for allowing material to be sprayed onto a surface such as the human skin are known, including aerosol devices for spraying wound care products onto wounds or burns. One such product is Savlon Dry (trade mark) which has been marketed in the UK by Zyma Healthcare and Ciba Geigy plc. Such products require the use of a gas propellant and in recent years the choice of gas propellants has become more limited because of the desire to avoid environmentally unfriendly compounds such as a chlorofluorocarbons or hydrocarbons. Also because small droplets and powder particles tend to be carried away from the target by the gas flow created when the propellant gas hits and is deflected by the target surface, such gas propelled sprays are generally designed to spray relatively large droplets or powder particles in order to achieve sufficient inertia to deposit the spray on its target surface. Such gas propelled products may run if sprayed too freely, especially where the spray produces large droplets. In addition, the packaging costs for such devices are high.

GB-A-1569707 describes a dispensing device for producing a spray or cloud of liquid droplets intended primarily for crop spraying. The process described in GB-A-1569707 produces liquid droplets by applying an electric field to a liquid emerging from an outlet in the vicinity of the surface so that the liquid becomes sufficiently charged that the net electric charge in the liquid as the liquid emerges into free space counteracts the surface tension forces of the liquid and the repulsive forces generated by the like electrical charges cause the liquid to be comminuted to produce a cone or jet which breaks into liquid droplets. The droplets produced by this device are charged close to their Rayleigh Limit and thus in use migrate quickly toward conductive surfaces of lower or zero potential. This technique of comminuting liquid is generally known as electrohydrodynamic comminution.

EP-A- 0468736 describes an electrostatic spraying device in which liquid emerging from an outlet of the device nozzle is subjected to an electrical field sufficiently high for the liquid to be drawn from the outlet as one or more ligaments which break up into charged droplets to form the spray. In order to provide shock suppression, the electrical field is produced by means of high voltage circuitry having a bi-polar output with a frequency no greater than 10 Hz.

According to the present invention, there is provided a method of providing a wound dressing, the method comprising the steps of:
supplying liquid to a liquid outlet in the vicinity of a substrate on which the wound dressing is to be provided;
subjecting liquid issuing from the liquid outlet to an electric field to cause the liquid to form at least one jet of electrically charged liquid, the liquid being such that, after formation, the at least one jet forms electrically charged fibre matter which is attracted to and deposits on the substrate to form a mat or web on the substrate; and
providing the mat or web with material for promoting or causing tissue repair.

The subjecting step may be repeated to provide the mat or web as a number of fibre matter layers.

The material may be provided by at least one of incorporating the material into the mat or web; incorporating the material into at least one fibre matter layer where the mat or web has a number of fibre matter layers; incorporating the material in the fibre matter; providing the material on the mat or web; providing the material on at least one fibre matter layer where the mat or web has a number of fibre matter layers; providing or interspersing the material between fibre matter layers where the mat or web has a number of fibre matter layers; providing the material on fibre matter during deposition; directing a spray containing the material onto the web or mat and/or at least one fibre matter layer where the mat or web has number of fibre matter layers and/or the fibre matter during deposition; providing the material within the liquid supply to the liquid outlet; electrohydrodynamically spraying a liquid comprising the material.

The material may comprise at least one of: cells for causing skin regrowth; skin cells; cytokines for promoting tissue repair or stimulating cytokinetic activity to promote cell activities to stimulate dendritic growth; material for promoting tissue repair to reduce scarring or otherwise control the sequence of events essential to natural tissue repair; growth factors such as fibroblast growth factor, epithelial growth factor, transforming growth factor.

The material may be provided by liquid which comprises an electret polymer; and the subjecting step may form electret polymer fibre matter which are attracted to and deposit onto the substrate to provide nuclei or otherwise initiate interactive cellular and/or molecular events in tissue repair.

The subjecting step may be carried out by using a hand holdable device comprising a housing having an opening registered with the liquid outlet and arranged to be positioned spaced from the substrate, for example 5 to 10 cm from the substrate, the housing being adapted to be held in the user's hand and the housing containing: a reservoir containing the liquid; a liquid supply tube for supplying liquid from the reservoir to the liquid outlet; and a voltage source which is activated by the user to cause a voltage to be applied to generate the electric field at the liquid outlet.

The subjecting step may cause the liquid issuing from the liquid outlet to form fibre matter having a diameter in the range of 10 nanometers to 10 micrometers.

At least one of the supply of liquid from the liquid outlet and the subjecting of the liquid issuing from the liquid outlet to the electric field may be controlled to cause the liquid to form electrically charged fibre matter having a diameter in the region of 10 nanometers to 10 micrometers and having a charge-to-mass ratio such that the fibre matter is attracted to and deposits onto the substrate to form to provide a controlled pattern of fibre matter.

The charge-to-mass ratio of the fibre matter may be controlled thereby enabling the location at which the fibre matter is deposited on the substrate to be controlled mainly by moving the liquid outlet relative to the substrate and controlling the number of passes and pattern of movement of the outlet over the substrate.

The mat or web may have adjacent fibre matter portions spaced apart by a gap.

In an embodiment, the subjecting step comprises regulating the flow of liquid to the liquid outlet to cause the liquid to control the diameter of the fibre matter.

The fibre matter may comprise ate least one of at least one fibre; fibre fragments or fibrils; particles.

In an embodiment, the subjecting step causes the mat or web to include, or provides in place of the mat or web, electrically charged fibre matter fragments which, in use, are electrically attracted to and stick into an area of skin or soft tissue to supply the material beneath the skin or into the soft tissue.

In an embodiment, the method further comprises changing the polarity of the electric field to change the polarity to which the fibre matter is charged to cause opposite polarity layers of fibre matter to be deposited in succession onto the substrate.

In an embodiment, the subjecting step subjects liquid issuing from the liquid outlet nozzle to an electric field while regulating the flow of liquid to the liquid outlet such that liquid issuing from the liquid outlet forms a multitude of liquid jets each of which forms fibre matter which is electrically attracted to and deposits onto the substrate.

In an embodiment, the substrate comprises a cavity or a concave surface and the method further comprises at least partially electrically discharging the electrically charged fibre matter prior to supply to the cavity or onto the concave surface.

The liquid may comprise a polymer, or solvent and polymer.

In an embodiment, the supplying step comprises supplying polymer containing liquid to the liquid outlet; the subjecting step forms electrically charged polymer fibre matter, and the method further comprises spraying the electrically charged polymer fibre matter with an oppositely charged spray or cloud. The polymer fibre matter may be sprayed with an oppositely charged spray comprising a surfactant,

In an embodiment, the subjecting step generates at least one electrically charged jet which partially solidifies to form electrically charged gel like fibre matter.

An embodiment further comprises causing the jet to break up to form fibre fragments by pulsing an applied voltage.

The substrate may be a wound dressing substrate or the wound itself.

Embodiments of the present invention will now be described, by way of example, with reference to the accompanying drawings, in which:
Figure 1 shows schematically one example of a device for carrying out a method embodying the invention;
Figures 2a to 2c are schematic diagrams for illustrating the mechanisms by which at least partially solid or gel-like particles, fibrils and fibres, respectively, may be produced by a method embodying the invention;
Figure 3 shows schematically another example of a device for carrying out a method embodying the invention;
Figure 4 shows schematically use of the device shown in Figure 3 to apply a dressing to the skin surface, a wound, burn or area exposed by a surgical procedure.
Figures 5 to 8 illustrate various different types of nozzles or outlets which may be used in a method embodying the invention;
Figure 9 shows a mat or web of fibres produced using a method embodying the present invention;
Figure 10 shows substantially parallel fibres deposited on a surface using a method embodying the present invention;
Figure 11 shows a part cross-sectional view of another example of a device for use in a method embodying the invention;
Figures 12 shows a nozzle which may be used to produce composite material; and
Figure 13 shows a nozzle for producing material from a mixture of two different liquids

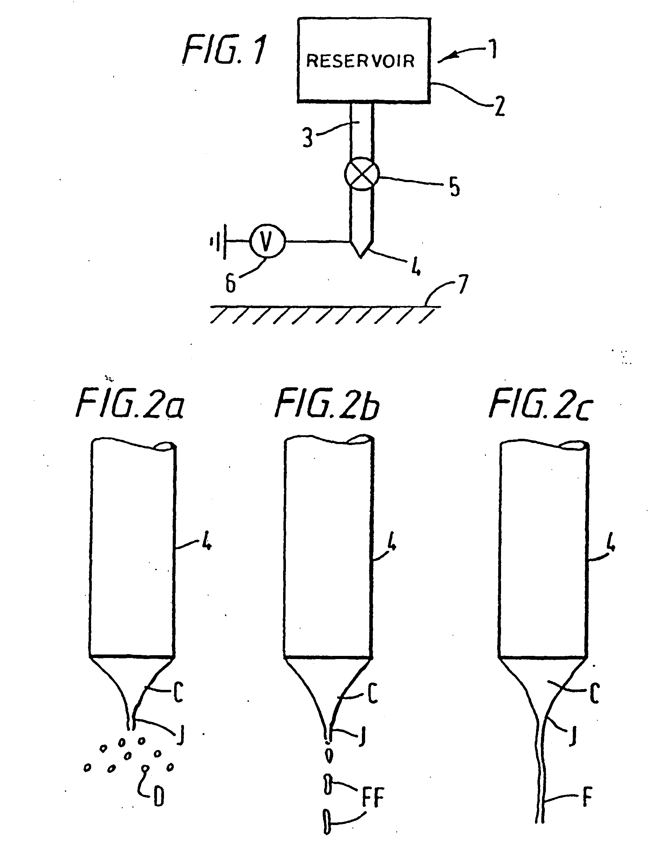

Referring now to the drawings, Figure 1 shows schematically apparatus 1 comprising a container or reservoir 2 of liquid coupled by a supply pipe 3 to an outlet 4 via a flow regulating valve 5 of conventional form. The valve 5 may be a manually or electrically operable valve. A voltage source 6 supplying a voltage of typically 15 to 25kV is coupled to the outlet 4 so as to cause liquid issuing from the outlet 4 to become charged. If the liquid is at least semiconducting (that is the liquid has a resistivity below about 10⁹ ohm-m), the voltage source 5 may be coupled to the liquid upstream of the outlet 4.

In use of the apparatus, a surface area 7 such as an area of the skin of an animal, for example an area of skin of a human being, is positioned a few centimetres, for example from 5 to 10 cm, below the outlet 4 as shown schematically in Figure 1. The voltage source 6 is coupled to the outlet 4 by closing a switch (not shown in Figure 1) and the flow regulating valve 5 opened so that liquid is supplied under gravity to the outlet 4. The liquid is selected to be biologically compatible, that is not harmful or detrimental to the animal when deposited on its skin or an open wound, and will typically have a resistivity in the range of from approximately 10² to 10⁸ ohm-metres and a viscosity in the region of from 0.1 to 1000 Poise or greater with the viscosity being dependent on whether a fibre, fibre fragments or segments or particles are to be formed.

As described in the aforementioned GB-A-1569707 and an article entitled "Electrodynamic Crop Spraying" by R. A. Coffee published in Outlook on Agriculture Volume 10 No. 7 1981, liquid issuing from the outlet 4 is subject to an intense electrical field which establishes a standing wave along the surface of the liquid producing cusps or cones which emit jets of charged liquid.

The small perturbations which inevitably occur in the liquid jet cause the jet to become unstable and the net electrical charge in the liquid provides a repulsive force which acts against the surface tension forces in the liquid. This would normally be expected, as described in GB-A-1569707, to cause the liquid to break up into droplets which, because both they and the outlet 4 are similarly charged, are propelled away from the outlet 4 and each other so providing a spray or cloud of liquid droplets. The present inventors have, however, found that by selecting the liquid and controlling the conditions of the electrohydrodynamic process, the jet of liquid, rather than breaking up into liquid droplets, forms a solid or gel-like fibre or forms fibre fragments (fibrils) or non-liquid droplets or particles. In use of the apparatus shown in Figure 1, the fact that the electrohydrodynamically produced material is charged and the animal body can effectively be considered earthed causes the material to deposit onto the surface 7 of the skin beneath the outlet 4. The material deposits swiftly, uniformly and gently by the energy contained in the electric field used to generate the material and will not overspray, nor become trapped in air streams and swept away from the target surface. One or more layers of such material may be deposited to provide a dressing to, for example, cover or protect, a wound or burn. This material being non-liquid should not cause the irritation which may arise from, for example, solvents if liquid droplets were applied to the skin.

Relative movement may be effected between the nozzle 4 and the surface 7, in this example the surface 7 may be moved, to enable coverage of a large area.

Figure 2a illustrates the situation where the liquid supplied to the outlet or nozzle 4 forms a spray of solid droplets or particles D while Figure 2b illustrates the situation where the liquid jet breaks up into fibrils FF and Figure 2c illustrates the situation where the liquid jet J forms a fibre F.

Figures 2a to 2c show only one cone C and the associated jet J emanating from a nozzle 4. The actual number of cone and jets produced will, however, depend upon several factors, including the resistivity, permittivity and flow rate of the liquid, the dimensions of the outlet 4 and the applied electric field.

In order to form the solid or gel-like droplets shown in Figure 2a, the liquid is selected or formulated so as to become non-liquid, that is at least partially solid or gel-like, after the liquid has been separated by the applied electric field into liquid droplets. Where the liquid includes a solvent, this may be achieved by, for example, selecting a liquid of such a volatility and viscosity and controlling the flow rate so that the solvent evaporates sufficiently to cause at least partial solidification or gellification only after droplet formation. Where the liquid is a melt which is held at an elevated temperature during supply to the outlet, then the liquid should be selected to have a melt temperature such that the liquid solidifies after liquid droplet formation. This may be facilitated by quenching using, for example, a cold inert gas or air stream.

To form the fibrils or fibre fragments shown in Figure 2b, the liquid is selected or formulated and the flow rate controlled so that the liquid jet becomes at least partially non-liquid, that is solid or gel-like, before the liquid has been separated by the applied electric field into liquid droplets but so that the growth wave resulting from perturbation of the jet J remains sufficiently strong to inhibit formation of a fibre and causes the jet to break up into fibre fragments or fibrils FF. This may be achieved by selecting the liquid and flow rate so that the liquid begins to solidify (for example by evaporation in the case of a solution or by cooling in the case of a melt) before droplet formation and becomes relatively brittle so that the growth wave causes the nascent fibre to break into segments. Break up of the nascent fibre into fibrils may be facilitated by pulsing the voltage applied to the outlet 4 so as the create an energy pulse which sets up a resonant process to promote breaking up of the nascent fibre. Experiments have shown that the length of the fibrils is related to the pulse duration or frequency with, under ideal conditions, the fibril length being equal to the jet velocity divided by the pulse frequency so that, for example, if the jet velocity is 5ms⁻¹ and a pulse frequency is 100kHz is used, the fibrils should have a length of 50µm. Fibrils having lengths in the region of, for example, tens of micrometres to a few centimetres may be produced, depending upon the particular liquid and electrohydrodynamic process conditions used.

In order to form the solid or gel-like fibre F shown in Figure 2c, the liquid is selected so as to become non-liquid, that is at least partially solid or gel-like, after issuing from the outlet, and the growth wave resulting from perturbation of the jet is attenuated so that the jet does not break up but forms a continuous fibre which has a length determined by the time for which the electrohydrodynamic process is continued, that is the time for which the voltage is applied. Attenuation of the growth wave may be achieved by the incipient solidification and/or by the nature of the liquid. Fibre, production may be achieved by, for example, selecting a liquid which is highly volatile or has a highly volatile component so that solidification by evaporation occurs very quickly before droplet formation. For example fibres may be formed using a liquid comprising a polymer which on solidification tends, because of its viscosity and/or polymer chain morphology, to resist growth wave development. Fibres may be formed using a relatively high molecular weight polymer, for example a polymer having a molecular weight in the region of 140000 or more. Where the liquid used is a melt then choosing a liquid which solidifies to a relativity plastic state should promote fibre formation.

The apparatus 1 shown in Figure 1 uses a gravity feed to supply liquids to the outlet 4 which has the advantage of simplicity. It is most suitable for use in situations where the area of skin to which the dressing is to be applied can easily be moved beneath the outlet 4 or for use when the liquid to be supplied may be detrimentally affected by pumping.

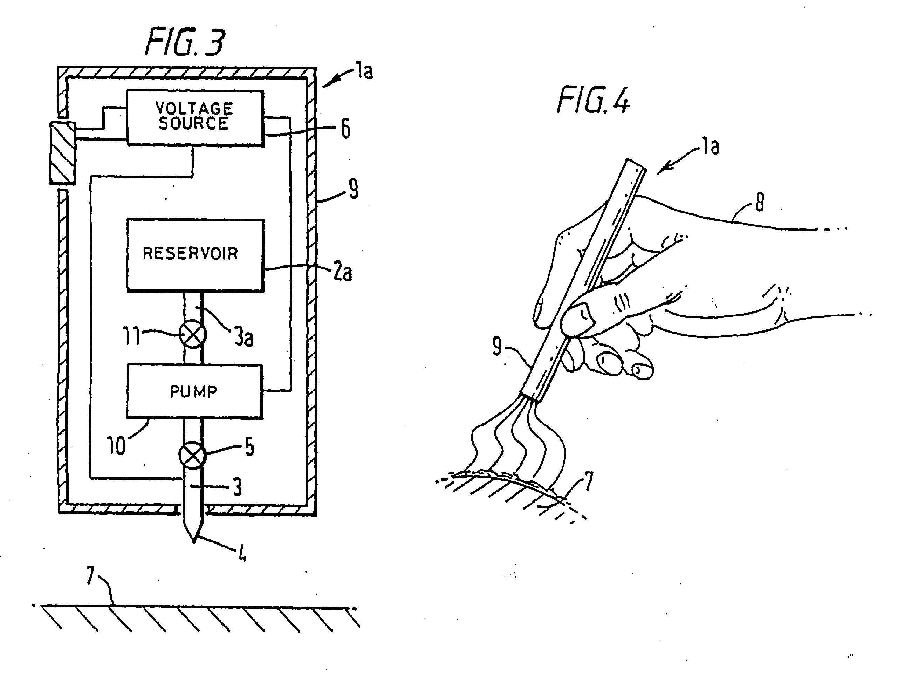

Figure 3 illustrates a part cross-sectional view of another form of apparatus 1a suitable for use in a method embodying the invention. The apparatus shown in Figure 3 is, as illustrated schematically in Figure 4, intended to be portable, in particular so as to be held in the hand 8 of a user.

The apparatus 1a shown in Figure 3 comprises a housing 9 within which is mounted a reservoir 2a of the liquid to be dispensed. The reservoir 2a may be formed as a collapsible bag so as to avoid any air contact with the liquid being dispensed. The reservoir 2a is coupled via a supply pipe 3a to a pump chamber 10 which is itself coupled via the supply pipe 3 and the flow regulating valve 5 to the outlet 4 in a similar manner to that shown in Figure 1. The voltage source 6 in this example is coupled to a user-operable switch SW1 which may be a conventional push button or toggle switch, for example. The voltage source 6 may comprise, for example a piezoelectric high voltage source of the type described in WO94/12285 or a battery operated electromagnetic high voltage multiplier such as that manufactured by Brandenburg, ASTEC Europe of Stourbridge West Midlands, UK or Start Spellman of Pulborough, West Sussex, UK and typically provides a voltage in the range of from 10 to 25kV. Although not shown, a voltage control circuit comprising one or more resistor capacitor networks may be provided to ramp the voltage up smoothly. The reservoir 2a may be coupled to the pump chamber 10 by way of a valve 11 which may be a simple non-return or one way valve or may be an electrically or mechanically operable valve of any suitable type, for example a solenoid or piezoelectric valve, operable by a voltage supplied by the aforementioned control circuit.

The pump chamber 10 may comprise any suitable form of pump, which provides a continuous substantially constant flow rate, for example an electrically operable pump such as a piezoelectric, or diaphragm pump or an electrohydrodynamic pump as described in EP-A-0029301 or EP-A-0102713 or an electroosmotic pump as described in WO94/12285 or a mechanically operable pump such as syringe pump operated or primed by a spring biassing arrangement operable by a user.

In use of the apparatus 1a shown in Figures 3 and 4, the user first positions the apparatus over the area 7 to which the material is to be applied, then actuates the switch SW1 and the pump of the pump chamber 10 to cause, when the valves 5 and 11 are opened, a stream of liquid to be supplied to the outlet 4 whence the liquid is subjected to the applied electric field as described above with reference to Figures 2a to 2c, forming charged matter which deposits onto the said surface 7 which may be the skin or on or within a wound. The user may move the apparatus or device 1a relative to the area 7 to cover a large area. One or more layers may be formed in a manner similar to that described with reference to Figure 1. The apparatus shown in Figures 3 and 4 has, however, the advantage that it is portable so allowing it to be used for, for example, first aid at the site of an accident and/or on relatively inaccessible areas of the body and does not rely on gravity fed.

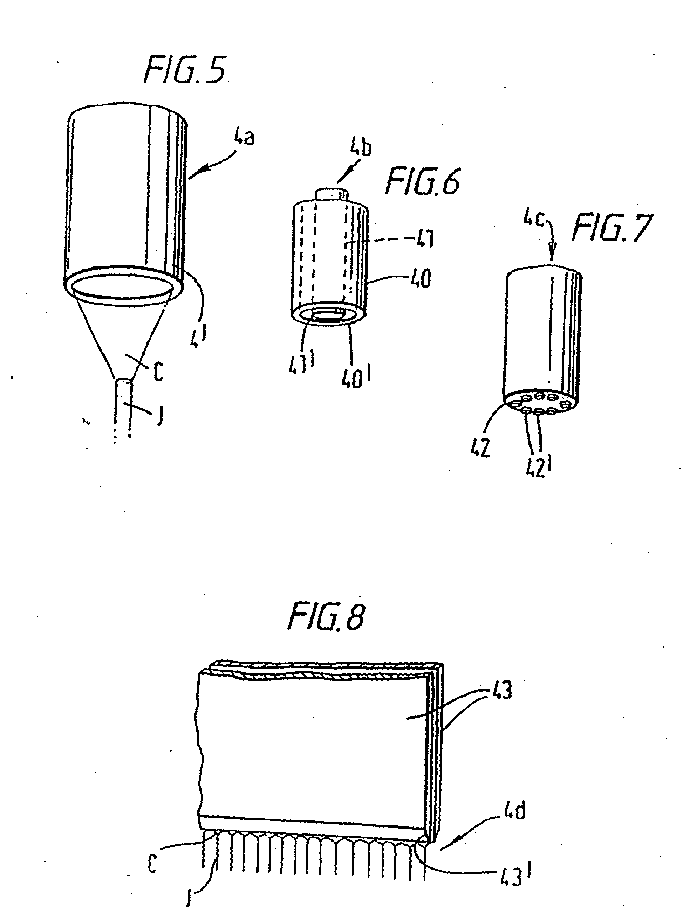

Various different forms of outlet or nozzle 4 may be used in the apparatus shown in Figures 1 and 3 and 4. Figures 5 to 8 illustrate schematically some examples. Another possibility is the fibre comminution site or nozzle described in WO95/26234.

The nozzle 4a shown in Figure 5 comprises a hollow cylinder which is conductive or semiconductive material at least adjacent its end 4' where the voltage is to be applied in use and will in use produce one or more jets (one cusp or cone C and jet J are shown) depending upon the resistivity and flow rate of the liquid and the voltage applied to the outlet 4.

The nozzle 4b shown in Figure 6 comprises two coaxial cylinders 40 and 41 at least one of which is conductive or semiconductive at least adjacent its end 40' or 41' where the voltage is applied and will in use produce a number of jets depending upon the resistivity and flow rate of the liquid and the applied voltage.

The nozzle 4c shown in Figure 7 comprises a number of parallel capillary outlets 42 which are conductive or semiconductive at least adjacent their ends 42' where the voltage is applied. Each capillary outlet 42 will normally produce a single jet. The multiple nozzles shown in Figure 7 have the advantage that blockage of one nozzle by relatively viscous liquid does not significantly affect the operation of the device and also allow different liquids to be supplied from respective reservoirs to different ones of the nozzles.

The nozzle 4d shown in Figure 8 comprises a slot-shaped nozzle defined between two parallel plates 43 which are conductive or semiconductive at least adjacent their ends 43' where the voltage is applied. The use of a slot nozzle when relatively highly viscous liquids are being used is advantageous because complete blockage of the nozzle is unlikely, as compared to the case where a relatively fine capillary nozzle is used, and a partial blockage should not significantly affect the functioning of the device because the liquid should be able to flow round any such partial blockage. The use of a slot-shaped nozzle outlet as shown in Figure 8 also allows a linear array of jets and thus of fibres, fibrils or particles or non-liquid droplets to be formed.

Where, as discussed above, the liquid being used is sufficiently conductive to enable the voltage to be applied to the liquid rather than the nozzle then the nozzle may be formed of any suitable electrically insulative material which does not retain electrical charge for any significant length of time, for example glass or a semi-insulating plastic such as polyacetyl.

The nozzle shown in Figure 7 is designed to produce a single jet per individual outlet 42. The nozzles shown in Figures 6 and 8 will in use produce a number of jets which extend generally along the electric field lines, with the number of jets depending upon, of course, the length of the slot (Figure 8) or the diameter of the annulus (Figure 6) and also upon the resistivity of the liquid, the flow rate and the applied voltage.

In the case of the cylindrical nozzle shown in Figure 5, when the flow rate is high only one jet will be produced as shown. However, at low flow rates, the liquid tends to emerge from the outlet as a film which clings to the rim of the cylinder and there forms multiple jets in a manner analogous to the annular nozzle shown in Figure 6.

Where the resistivity of the liquid is high, for example about 10⁹ ohm-m, some 10 or 20 jets, dependent upon the applied voltage and flow rate, may be formed per cm length of the nozzle, allowing the same number of fibres, for example, to be produced ("spun"). The applied voltage also affects the diameter of the resulting material. Thus, about 10 to 15 fibres of about 10 to 20 micrometres in diameter may be formed per cm length of the slot shown in Figure 8 from a liquid having a resistivity of about 10⁹ ohm-m when the applied voltage is 15 kilovolts and a larger number, about 20, of fibres of smaller diameter may be formed per cm length of the slot when the applied voltage is 25 kilovolts. At liquid resistivities of, for example, 10⁷ ohm-m, some 5 to 10 fibres may be spun per cm length of the slot, dependent again on the applied voltage and flow rate, with again a larger number of thinner fibres being formed at higher voltages. The number of jets produced decreases but their diameter increases with increasing flow rate. By selecting the resistivity and viscosity of the liquid, the flow rate and the applied voltage, material, for example fibres or fibrils, with diameters from a few, about 10 nanometres (nm) to above 100 micrometres, typically 10² to 10⁴ nm, may be produced. Similar results may be achieved using the hollow cylinder nozzle of Figure 5 or the annular nozzle of Figure 6.

The use of a liquid which is controlled to produce fibres is particularly advantageous for producing a wound or burn dressing because, as will be described below, deposition of the fibres onto the area being covered results in a network of crossing or interlinking fibres providing effectively an integral web or mat which has a high specific surface area and is thus highly absorbent to fluids, whilst being exceptionally light. Like a conventional dressing it enables good coverage over an area of skin so as, for example, to protect a wound but, unlike many conventional dressings, still enables, by virtue of the gaps between the network of fibres, air to pass through the dressing to the wound and pus and other detritus to pass from the wound, while preventing ingress of bacterial matter into the wound.

By controlling the diameters of the fibres in the manner described above and/or by controlling the number of layers of fibres, dressings having a range of thickness, fluid permeability and mechanical strength can be formed enabling the dressing to be adapted for use on different types of wounds and burns including wounds arising from severe trauma such as say motor vehicle accidents, battle wounds etc, and chronic wounds including lesions such as ulcerated veins as well as, where appropriate, surgically exposed tissue. The permeability of the dressing has been found to be a function of the diameters and spacing of the fibres and the motion of the nozzle over the deposition area during application.

Liquids which form short fibrils or solid droplets will not generally form a cohesive mat or web of fibres. However, liquids which form fibrils or solid droplets may be used in combination with conventional dressings or with dressings formed by fibres as discussed above, for example fibrils or solid droplets produced using a method embodying the invention may be deposited into or on a wound and then covered with one or more layers of fibres produced by method embodying the invention or by a conventional dressing.

Fibres, fibrils or droplets produced by a method embodying the invention may be deposited onto a substrate, such as a dressing, for later application to the skin, a wound, burn or the like.

Experiments have been carried out with a number of different polymers and solvents. It has been found that long chain heavy molecular structures facilitate fibre production while short chain length molecular structures tend to form fragments or solid droplets. Solvents which evaporate quickly during the jet flow may be used to facilitate formation of fibres. Suitable solvents may be, for example, methanol, propanol and water, methylene chloride, acetone and chloroform, depending upon the particular polymer used.

Experiments have been carried out in which the apparatus shown in Figure 1 was used with water and hydrocarbon based solutions supplied to a slot-like nozzle of the type shown in Figure 8 having a slot width of about 150 micrometres and a slot length of 2cm. Liquid flow rates of from 1 to 10 microlitres per second and voltages of from 10kV to 15kV were found to produce about 5 to 15 charged fibres per cm length of the slot with the fibres having diameters in the range of from 1 to 100 micrometres.

Fibres have been successfully spun with polyhydroxybutyric acid, a bioresorbable polymer, and polyvinyl alcohol (PVA), a polymer soluble in water and alcohols such as methanol or propanol, and pharmaceutical preparations for wound care, such as "New Skin" (trade mark) marketed by SmithKline Beecham which comprises nitrocellulose in an organic solution (in particular it comprises ethyl acetate, isopropyl alcohol, amyl acetate, isobutyl alcohol, denatured alcohol, camphor and nitrocellulose). "New Skin" is normally applied to scratches and light wounds with a rod or paddle because it is too viscous to be applicable by conventional spray devices. "New Skin" has however been successfully sprayed by a method embodying the invention to form fibres of approximately 0.5 to 5 micrometres diameter which deposited uniformly onto skin, resulting in a firm skin-like web-film. In one specific example neat (that is undiluted) "New Skin" was supplied at a flow rate of 4 millilitres per hour to a capillary nozzle of the type shown in Figure 5 in the form of a 1.1mm diameter thin-walled metal, generally stainless steel, tube. A voltage of 8.2kV was applied to the nozzle which was located approximately 50mm above an earthed deposition surface. Multiple fibres were formed and substantially uniformly deposited on the surface. Fibres have also been produced using undiluted "New Skin" (trade mark) with flow rates of from 1 millilitres per hour to 100 millilitres per hour.

Polyvinyl alcohol (PVA) has also been deposited in a similar manner to the "New Skin", using combinations of alcohol and water as solvent. Neat, undiluted PVA having a molecular weight of typically 15000 has been found to tend to form solid droplets when electrohydrodynamically processed while PVA having a molecular weight of about 140000 or more tends to form fibres. Low molecular weight PVA in a volatile solvent such as ethanol tends to break up into fibrils rather than continuous fibres. Thus, PVA having a molecular weight in the region of about 90000 to 140000 will tend to form fibrils and PVA fibrils having diameters of a few hundred nanometres and lengths of 0.5 to 10mm have been produced.

In another experiment, an annular nozzle of the type shown in Figure 6 was used to which a voltage of from 5kV to 15kV was applied. A 90% by volume solution of poly β-hydroxybutric acid (which is a bioresorbable polymer) in methylene chloride was supplied at a flow rate of from 5 micro litres per second to 50 micro litres per second to the nozzle which was located at a distance of about 5cm from human skin. A covering layer of fibres was formed on the skin with the fibres having diameters, dependent on the applied voltage and flow rate, in the range of from about 10 micrometres to about 50 micrometres.

In another example, the apparatus shown in Figure 1 was used with a thin-walled, generally stainless steel, capillary nozzle of the type shown in Figure 5 having a 1.1mm external diameter. The reservoir was filled with polylactic acid having a molecular weight of 144000 dissolved 10% by mass in acetone and the flow regulator was controlled to provide a flow rate of 10 millilitres per hour. A voltage of 12 kV was applied to the nozzle which was located 8cm away from and perpendicular to a flat earthed counter electrode provided to simulate a skin surface. This experiment was also repeated using a flow rate of 6.0 millilitres per hour and a nozzle voltage of 11.4kV. The surface of the flat plate was covered by a network or mass of randomly distributed fibres having diameters typically in the region of from 2 micrometres to 7 micrometres.

The fibres deposit readily onto capacitive or earthed surfaces without any of the normal problems of applying very low mass high specific surface materials and the electrical field ensures that the fibres deposit swiftly, gently and substantially uniformly.

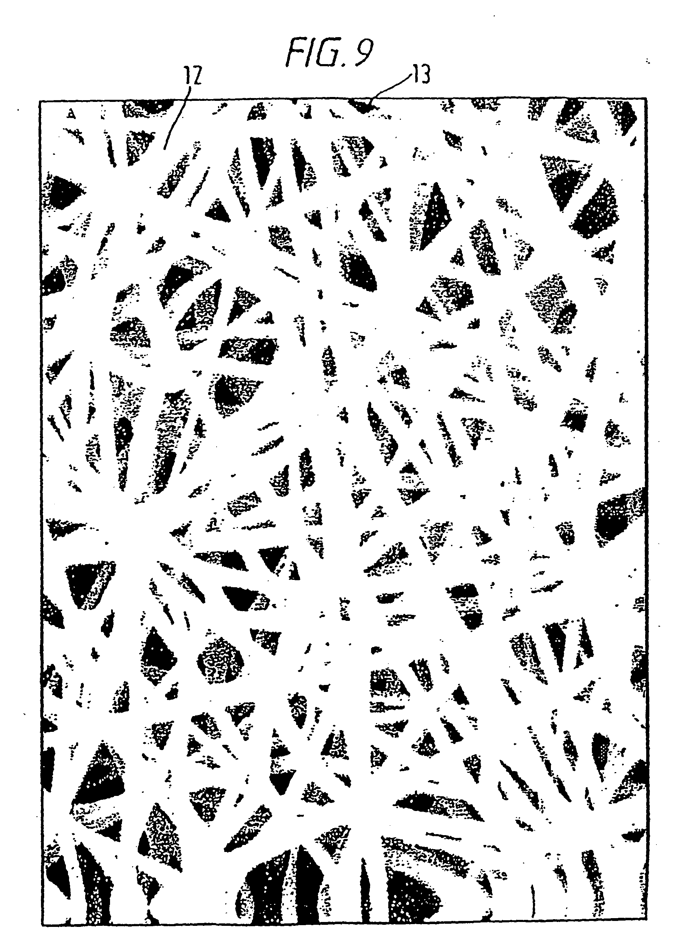

Figure 9 shows a copy of an image produced by scanning electron microscope of a typical mat or web of fibres 12 on a plate 13. The fibres have, typically, a diameter of approximately 5µm. The fibres shown in Figure 9 are relatively randomly distributed because their relatively low mass, and thus low inertia, and high charge to mass ratio means that their movement and thus location of deposition on the surface is strongly influenced by the fact that they are all similarly charged fibres. This also results in the fibres crossing one another and possibly even blending together which should increase the overall mechanical integrity of the web or mat.

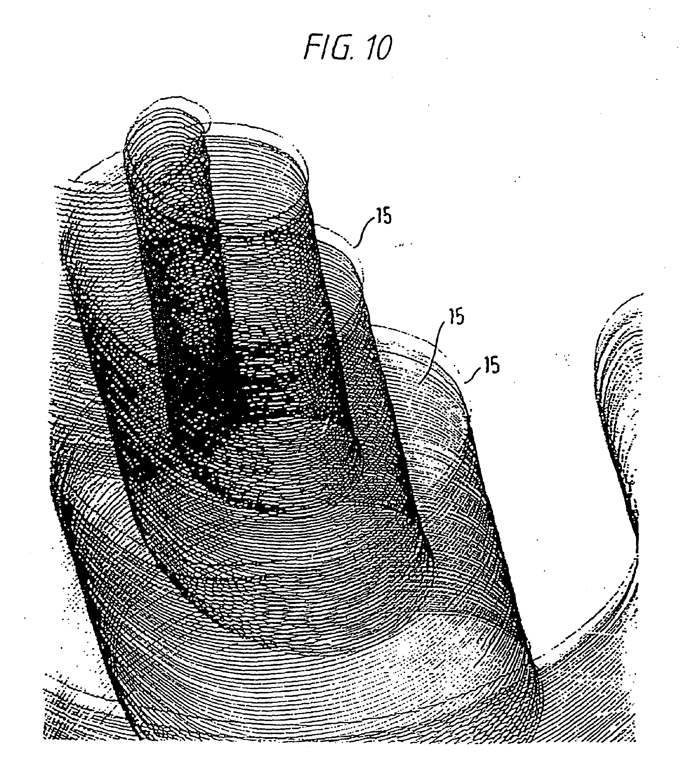

By increasing the mass of the fibres and thus their inertia, and reducing their charge to mass ratio, greater control can be achieved over the deposition of the fibres so that the location at which the fibres are deposited on the skin or wound can be controlled mainly by moving the nozzle relative to the skin or wound and by controlling the number of passes and pattern of movement of the nozzle over the surface. Figure 10 shows an example of fibres 15 of about 50 to 100 micrometres in diameter deposited onto a substrate using a slot-shaped nozzle of the type shown in Figure 8. As can be seen from Figure 10, a single pass of the nozzle produces a set of approximately parallel tracks and, with two or more passes, a relatively dense material akin to a textile can be produced. Although the actual pattern shown in Figure 10 was produced by depositing fibres of a heavy build viscous paint onto paper, it will be appreciated that similar results can be achieved using other material such as inert polymers of similar mass. The movement of the nozzle may be controlled to produce any desired pattern and that for example a woven texture could be simulated. Such fibres may be used, for example to form a bandage.

In the examples described above, the fibres, fibrils or droplets produced using the method embodying the invention consist simply of an inert polymer which may be a bioresorbable polymer such as polyhydroxybutyric acid, polyvinyl alcohol, polyglycolic acid or polylactic acid. Biologically active ingredients may, however, be added to the liquid before it is supplied to the outlet nozzle 4. In such cases, the liquid may comprise a solution, suspension, microsuspension, emulsion, microemulsion, gel or even a melt containing the active component or components. Possible active components are one or more of the following, namely pharmaceutical compounds such as analgesics, antiseptics, antibiotics, bactericides, antifungals, antiparasitics, antiinflammatory agents, vasodilators (such as minoxidil which is believed to promote wound epithelialization and neovascularization), agents such as proteolytic enzymes for debridement and tissue repair promoting materials such as for example cytokines for stimulating cytokinetic activity to promote essential cell activities, for example to stimulate dendritic growth, growth factors such as fibroblast growth factor (FGF), epithelial growth factor (EGF), transforming growth factor (TGF) that are believed to reduce scarring and others that may be used to promote or otherwise control the sequence of events essential to natural tissue repair, cells, peptides, polypeptides, insulin, immune suppressants or stimulants and vaccines. Another possible active components are DNA or other genetic matter for gene therapy, surface binding or surface recognising agents such as surface protein A, and surfactants.

Where more than one layer of fibres, fibrils and/or particles is deposited, then different active ingredients may be provided in the different layers and different biologically active ingredients may be included in different fibres, fibrils or particles where a nozzle of the type shown in Figure 7 is used. Also biologically active ingredients may be provided between layers, for example skin cells may be interspersed in or between layers.

The active ingredient may comprise an adjuvant that is a pharmacological agent added to a drug to increase or aid its effect or an immunological agent that increases the antigenic response.

Where the resulting material is in a form of fibrils, the fibrils may actually stick into the surface, for example skin or soft tissue, onto which they are deposited so enabling, for example, the supply of drugs and other biologically active agents beneath the skin or into the soft tissue, and may for example be used to carry DNA to cells.

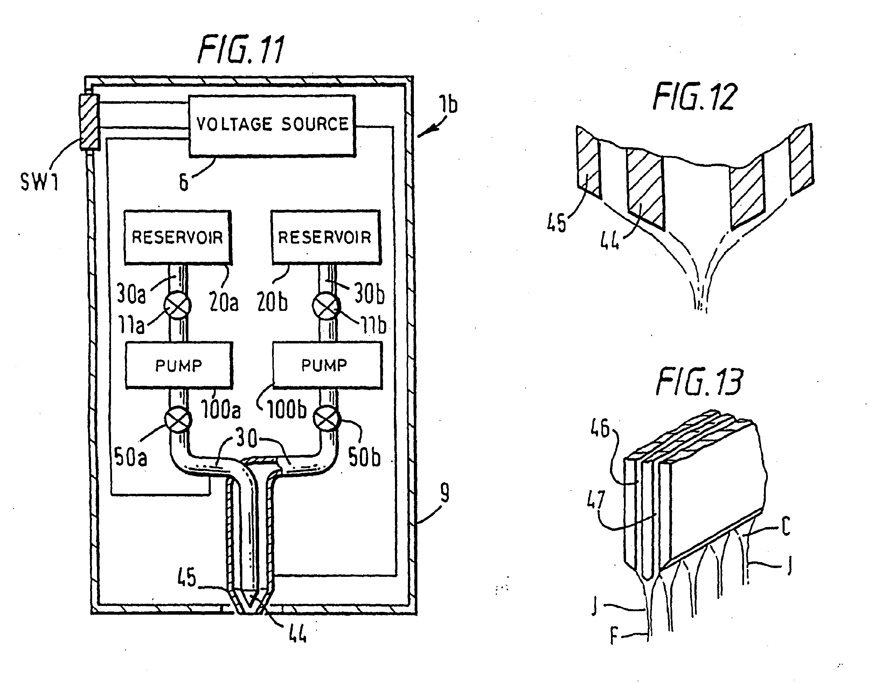

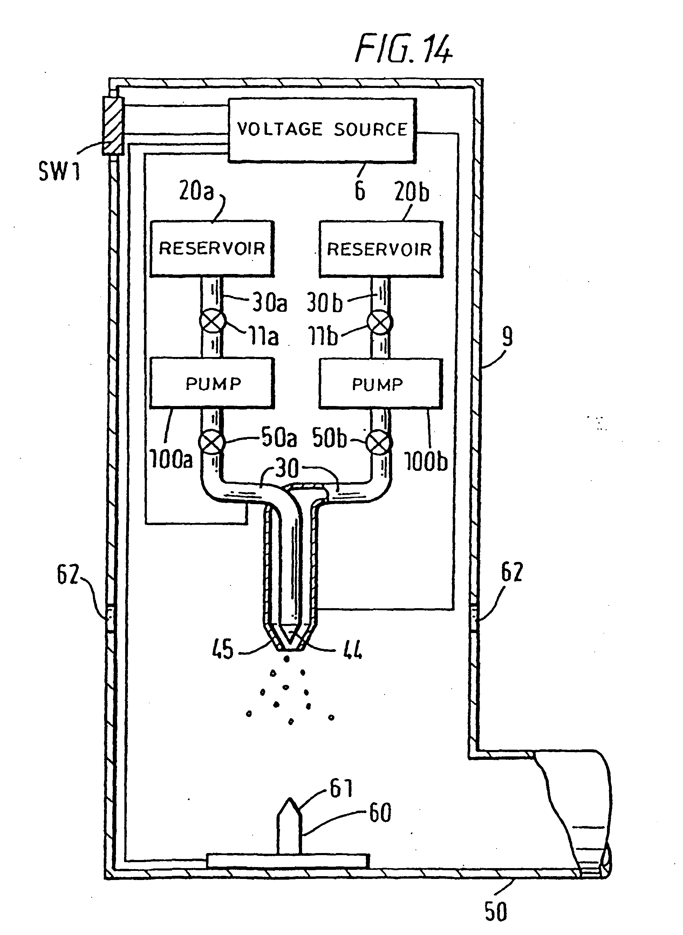

Figure 11 illustrates a modified form of the device shown in Figure 3. The device 1b shown in Figure 11 is essentially similar to that shown in Figure 3 but comprises two reservoirs 20a and 20b each coupled by respective supply pipes 30a and 30b and possibly by non-return valves 11a and 11b to a respective pump chamber 100a and 100b coupled via a respective valve 50a and 50b to a respective liquid supply pipe 30 which terminates in a respective outlet 44 and 45 arranged so that the outlet 45 is coaxial with and extends around the outlet 44. Figure 12 shows the outlets 44 and 45 on an enlarged scale. The device 1b shown in Figure 11 allows different forms of liquid to be supplied to the electrohydrodynamic processing site provided by the outlets 44 and 45.

The reservoir 20a coupled to the inner outlet 44 may contain a supply of a biologically active ingredient such as a pharmaceutical or a solution of DNA for example, while the reservoir 20b coupled to the outer nozzle 45 may contain a supply of a polymer solution of the type discussed above, for example polyhydroxybutyric acid dissolved in methylene chloride. The device shown in Figure 11 is operated in a similar manner to the device shown in Figure 3. Thus, the switch SW1 is first activated to supply the required voltages, typically 10 to 25kV, the flow regulating valves 50a and 50b are then opened to provide the required flow from each of the nozzles 44 and 45 and the pumps 100a and 100b and valves 11a and 11b, if present, activated to supply liquid to the respective nozzles 44 and 45. The outlets of the two coaxial nozzles are designed to promote laminar flow so that the polymer containing solution issues from the nozzle 45 so as to surround the other liquid.

By appropriate selection the molecular weight of the polymer and/or the volatility of the polymer solution, the liquids issuing from the combined nozzle can be caused to form a fibre or fibrils in which the biologically active ingredient forms a cylindrical centre core of the fibre or fibril or micro capsules in which the biologically active ingredient is completely encapsulated within the polymer and may still be in a liquid form.

For microcapsule formation it has been found preferable to reduce the percentage polymer in solution and to use a much reduced molecular weight polymer. For example a resin such as neoprene chlorinated rubber dissolved at more than about 10% by weight in trichloroethane will tend to spray fibres. However by decreasing the percentage polymer and/or using a less volatile solvent such as, for example, propylene glycol ether, microcapsules may be formed. Microcapsules have been produced using PVA of low molecular weight, for example a molecular weight of about 15000, dissolved to a dilution of between about 2.5 per cent and 5 per cent by volume in water or alcohol with a flow rate of about 1.0 microlitres per second. Production of microcapsules may be enhanced by using two reactive monomers one of which is placed in each of the two liquids to react during comminution.

The composite products produced using the device shown in Figures 11 and 12 may be used to form a dressing in the manner described above where the composite product is in the form of fibres or long fibrils allowing for controlled release of the active ingredient as the bioresorbable polymer degrades. Where the composite products produced are fibres, fibrils or microcapsules, then these may be applied to the surface of the skin or into a wound in combination with, for example, a conventional dressing or a dressing produced from comminuted fibres. Material from the core of a fibre or fibril may be released from the ends of the fibre or fibril. Material from the core of a fibre, fibril or microcapsule may be released through the coating if the coating is permeable to the material contained within it or may be released as a result of the outer coating being breached, for example by chemical or enzymic attack which causes the outer coating to dissolve or degrade, by bioresorption or biodegradation of the coating, or as a result of temperature changes or application of pressure which causes the outer coating to rupture.

Composite products made up of three or more different layers of material may be formed by increasing the number of coaxial nozzles.

The outlet nozzle of the device shown in Figure 11 may comprise a number of sets of coaxial outlet nozzles 44 and 45 in a manner similar to that shown in Figure 7 for single outlet nozzles. This would allow different active ingredients to be supplied to different ones of the inner nozzles 44. The different active ingredients can thus be kept apart until actual use which is of particular advantage where the active ingredients react to form a product which itself has a low shelf life.

It will, of course, be appreciated that the apparatus shown in Figure 1 could be modified in a manner similar to that shown in Figure 11 for Figure 3 to produce a device capable of forming cored fibres, fibrils or microcapsules.

As discussed above, the nozzle shown in Figure 12 is deliberately designed to avoid mixing between the two liquids which are generally selected so as to be immiscible thereby enabling production of a cored fibre, fibril or microcapsule.

Figure 13 shows an alternative form of nozzle which may be used in the apparatus shown in Figure 11. The nozzle shown in Figure 13 is a slot-nozzle similar to that shown in Figure 8 but provided with two separate channels 46 and 47 coupled to respective ones of the liquid supply pipes so that each channel receives a different liquid. The outlets of the channels 46 and 47 are designed so as to create turbulence and therefore mixing of two liquids at the outlet. This arrangement may be used where, for example, it is desired to have some control over the amount of active ingredient which may be incorporated into a liquid or to combine two liquids which then react. A polyurethane foam has been formed by reacting a solution of urethane supplied via one of the nozzles with a blowing agent supplied by the other nozzle to spray a flexible foam deposit into a wound to form a cavity wound dressing. This arrangement has the advantage that the dressing will conform to the contours of a cavity wound and may be applied with clerical cleanliness without handling. Again, an active ingredient such as a pharmaceutically active ingredient may be incorporated into one of the two liquids or mixed with the two liquids.

The nozzle shown in Figure 13 may also be used to, for example, bring reactive liquids together at the nozzle to deposit reacting or reactive product onto the skin or into a wound which should be of advantage where the reactive product has a very short lifetime and cannot be stored. For example, the nozzle shown in Figure 13 has been used experimentally to produce a fibrin mat by supplying the enzyme thrombin to one channel and fibrinogen to the other channel.

As another possibility the device shown in Figure 11 may be modified to provide two separate spaced nozzles and the voltage source arranged to charge the two nozzles to voltages of opposite polarity in a manner similar to that described in WO94/12285 so as to enable liquid droplets charged to one polarity to rapidly coalesce with droplets charged to the other polarity to form ultra-small particles of from sub-micron to a few tens of microns in diameter. Again, for example, ultra small droplets containing, for example the enzyme thrombin may be sprayed at one polarity so as to rapidly coalesce with droplets of the opposite polarity containing fibrinogen to deposit a fast reacting fibrin mat to cause blood clotting, for wound sealing or for adhesion.

Material capable of transfecting resident cells in situ with genetic material in order to regulate cell responses may be produced. For example microcapsules comprising DNA encapsulated in a microcapsules or complexed with an appropriate lipid material for transecting cells may be produced. Phospholipid microcapsules encapsulating DNA may be produced by a method embodying the invention. Other biological material such as proteins may be similarly encapsulated or complexed with an appropriate lipid material. Proteins may also be incorporated in the lipid layer. Surface binding or surface recognising agents such as surface protein A may be incorporated into microcapsules, especially phospholipid microcapsules, for selecting targets such as cancer cells, epithelial cells etc. Also, surfactants such as soya lecithin available from Sigma Pharmaceuticals may be incorporated in the outer surface of fibres, microcapsules or fibrils.

Fibres, fibrils or droplets or capsules may be coated with substances such as surfactants such as soya lecithin or with, for example, DNA which is relatively sticky. This may be achieved by, for example, supplying the polymer containing liquid to the inner nozzle in Figure 11 and supplying the coating material to the outer nozzle in Figure 11. Alternatively, a separate spraying device, which may be a conventional or electrohydrodynamic spraying device, may be provided so as to direct, for example, an oppositely charged spray or cloud of the coating material into the path of the material produced by the apparatus shown in Figure 1, 2 or 11, for example.

Where a method embodying the invention is used to produce fibres, fibrils or microcapsules comprising a core of an active ingredient, the choice of coating material, the permeability and/or thickness of the coating may be justed to adjust the timing of release of the active ingredient. For example where the coating comprises a bioresorbable or biodegradable polymer, the half-life of the polymer may be controlled by controlling the permeability and/or thickness of the polymer coating by, for a specific formulation, controlling the flow rate and voltage.

Materials formed of two or more components which have only a short-shelf life when mixed together may be formed in a timely manner by encapsulating the respective components in respective fibres, fibrils or microcapsules so that mixing of the various components only occurs when the components are released from the encapsulating material by, for example, leaching through the encapsulating material, rupture by pressure being applied to the encapsulating material, temperature, or degradation, for example bioresorption or biodegradation, of the encapsulant.

Generally the capacitive nature of materials such as skin and the moisture content of the air should be sufficient for deposition onto a surface to occur simply by electrostatic attraction. However, where it is desired to deposit a large amount of material, then it may be necessary to earth the surface or to maintain it at a lower or opposite potential to the charged matter.

Material may be supplied to cavities and concave surfaces. In such circumstances, the charged matter will generally be at least partially electrically discharged before it reaches the cavity or concave surface.

As used herein, the term "particle" includes solid, partially solid and gel-like droplets and microcapsules which incorporated solid, partially solid, gel-like or liquid matter. The term "active ingredient" means material which is compatible with and has an affect on the substrate or body to which it is to be applied and the term "biologically active ingredient" or "biologically active material" means material which is compatible with and has an affect (which may for example be biological, chemical or biochemical) on the animal or plant to which it is to be applied and includes, for example, medicaments such as proprietary medicines, pharmaceutical medicines and veterinary medicines, vaccines, genetic material such as DNA, cells and the like.

## Claims

1. A method of providing a wound dressing, the method comprising the steps of:
supplying liquid to a liquid outlet (4) in the vicinity of a substrate on which the wound dressing is to be provided;
subjecting liquid issuing from the liquid outlet (4) to an electric field to cause the liquid to form at least one jet of electrically charged liquid, the liquid being such that, after formation, the at least one jet forms electrically charged fibre matter which is attracted to and deposits on the substrate to form a mat or web on the substrate; and
providing the mat or web with material for promoting or causing tissue repair.

2. A method according to claim 1, which comprises repeating the subjecting step to provide the mat or web as a number of fibre matter layers.

3. A method' according to Claim 1 or 2, which comprises providing the material by at least one of: incorporating the material into the mat or web; incorporating the material into at least one fibre matter layer where the mat or web has a number of fibre matter layers; incorporating the material in the fibre matter; providing the material on the mat or web; providing the material on at least one fibre matter layer where the mat or web has a number of fibre matter layers; providing or interspersing the material between fibre matter layers where the mat or web has a number of fibre matter layers; providing the material on fibre matter during deposition; directing a spray containing the material onto the web or mat and/or onto at least one fibre matter slayer where the mat or web has number of fibre matter layers and/or onto the fibre matter during deposition; providing the material within the liquid supply to the liquid outlet; electrohydrodynamically spraying a liquid comprising the material.

4. A method according to any of the preceding claims, wherein the material comprises at least one of: cells for causing skin regrowth; skin cells; cytokines for promoting tissue repair or stimulating cytokinetic activity to promote cell activities to stimulate dendritic growth; material for promoting tissue repair to reduce scarring or otherwise control the sequence of events essential to natural tissue repair; a growth factor; fibroblast growth factor, epithelial growth factor, transforming growth factor.

5. A method according to any of claims 1 to 3, wherein the liquid comprises an electret polymer; and the subjecting step forms electret polymer fibre matter which are attracted to and deposit onto the substrate to provide nuclei or otherwise initiate interactive cellular attd/or molecular events in tissue repair.

6. A method according to any preceding claim, which comprises carrying out the subjecting step by using a hand holdable device (1a) comprising a housing (9) having an opening registered with the outlet (4) and arranged to be positioned spaced from the substrate (7), the housing (a) being adapted to be held in the user's hand and the housing containing: a reservoir (2a) containing the liquid; a liquid supply tube for supplying liquid from the reservoir to the liquid outlet (4); and a voltage source (6) which is activated by the user to cause a voltage to be applied to generate the electric field at the liquid outlets (4).

7. A method according to claim 6, wherein are housing opening is positioned spaced 5 to 10 cm from the substrate.

8. A method according to any preceding claim, wherein the subjecting step causes the liquid issuing from the liquid outlet (4) to form fibre matter halving a diameter in the range of 10 nanometers to 10 micrometers.

9. A method according to any of claims 1 to 7, further comprising controlling at least one of the supply of liquid from the liquid outlet (4) and the subjecting of the liquid issuing from the liquid outlet (4) to the electric field to cause the liquid to form electrically charged fibre matter having a diameter in the region of 10 nanometers to 10 micrometers and having a charge-to-mass ratio such that the fibre matter is attracted to and deposits onto the substrate to form to provide a controlled pattern of fibre matter.

10. A method according to any of claims 1 to 8, which comprises controlling the charge-to-mass ratio of the fibre matter thereby enabling the location at which the fibre matter is deposited on the substrate to be controlled mainly by moving the liquid outlet (4) relative to the substrate and controlling the number of passes and pattern of movement of the outlet over the substrate.

11. A method according to any preceding claim, wherein the mat or web has adjacent fibre matter portions spaced apart by a gap.

12. A method according to any preceding claim, wherein the subjecting step comprises regulating the flow of liquid to the liquid outlet (4) to cause the liquid to control the diameter of the fibre matter.

13. A method according to any of the preceding claims, wherein the fibre matter comprises at least one of : at least one fibre; fibre fragments or fibrils; particles

14. A method according to any of claims 1 to 12, wherein the subjecting step causes the mat or web to include, or provides in place of the mat or web, electrically charged fibre matter fragments which in use, are electrically attracted to and stick into an area of skin or soft tissue to supply the material beneath the skin or into the soft tissue.

15. A method according to any preceding claim, further comprising changing the polarity of the electric field to change the polarity to which the fibre matter is charged to cause opposite polarity layers of fibre matter to be deposited in succession onto the substrate.

16. A method according to any preceding claim, wherein the subjecting step subjects liquid issuing from the liquid outlet (4) to an electric field while regulating the flow of liquid to the liquid outlet (4) such that liquid issuing from the liquid outlet (4) forms a multitude of liquid jets each of which forms fibre matter which is electrically attracted to and deposits onto the substrate.

17. A method according to any of the preceding claims, wherein the substrate comprises a cavity or a concave surface and the method further comprises at least partially electrically discharging the electrically charged fibre matter prior to supply to the cavity or onto the concave surface.

18. A method according to any preceding claim, wherein the liquid comprises a polymer, or solvent and polymer.

19. A method according to any of claims 1 to 17, wherein: the supplying step comprises supplying polymer containing liquid to the liquid outlet (4); the subjecting step forms electrically charged polymer fibre matter; and the method further comprises spraying the electrically charged polymer fibre matter with an oppositely charged spray or cloud.

20. A method according to claim 18 which comprises spraying the polymer fibre matter with an oppositely charged spray comprising a surfactant.

21. A method according to any of the preceding claims, wherein: the subjecting step generates at least one electrically charged jet which partially solidifies to form electrically charged gel like fibre matter.

22. A method according to any-preceding claim, further comprising causing the jet to break up to form fibre fragments by pulsing an applied voltage.

23. A method according to any of the preceding claims, wherein the substrate is either a wound dressing substrate or the wound itself.

## Patentansprüche

1. Verfahren zur Herstellung eines Wundverbands, wobei
einem Flüssigkeitsauslass (4) in der Nähe eines Substrats, auf dem der Wundverband erzeugt werden soll, Flüssigkeit zugeführt wird,
die an dem Flüssigkeitsauslass (4) austretende Flüssigkeit mit einem elektrischen Feld beaufschlagt wird, so dass die Flüssigkeit mindestens einen Strahl aus elektrisch geladener Flüssigkeit bildet, wobei die Flüssigkeit so beschaffen ist, dass der mindestens eine Strahl nach seiner Ausbildung einen elektrisch geladenen Faserstoff erzeugt, der von dem Substrat angezogen wird und sich unter Bildung einer Matte oder Bahn auf dem Substrat ablagert, und
die Matte oder Bahn mit einer Substanz versehen wird, die die Gewebewiederherstellung fördert oder bewirkt.

2. Verfahren nach Anspruch 1, wobei die Beaufschlagung mit dem elektrischen Feld wiederholt wird, um die Matte oder Bahn in mehreren Faserstoffschichten zu erzeugen.

3. Verfahren nach Anspruch 1 oder 2, wobei die Substanz dadurch vorgesehen wird, dass sie in die Matte oder Bahn oder, soweit diese mehrere Faserstoffschichten aufweist, in mindestens eine Faserstoffschicht oder in den Faserstoff eingebaut wird, oder auf der Matte oder Bahn oder, soweit diese mehrere Faserstoffschichten aufweist, auf mindestens einer Faserstoffschicht vorgesehen wird, oder, soweit die Matte oder Bahn mehrere Faserstoffschichten aufweist, zwischen die Faserstoffschichten eingebracht wird, oder während des Auftrags auf Faserstoff vorgesehen wird, oder ein die Substanz enthaltender Sprühnebel auf die Bahn oder Matte und/oder, soweit diese mehrere Faserstoffschichten aufweist, auf mindestens eine Faserstoffschicht oder während des Auftrags auf den Faserstoff gerichtet wird, oder in die dem Flüssigkeitsauslass zugeführte Flüssigkeit eingebracht wird, oder eine die Substanz enthaltende Flüssigkeit elektrohydrodynamisch versprüht wird.

4. Verfahren nach einem der vorhergehenden Ansprüche, wobei die Substanz mindestens einen der folgenden Inhaltsstoffe enthält: Zellen, die die Nachbildung von Haut bewirkt; Hautzellen; Cytokine zur Förderung der Gewebewiederherstellung oder Stimulation cytokinetischer Aktivität zur Förderung der Zellaktivität, um dentritisches Wachstum anzuregen; eine Substanz zur Förderung der Gewebewiederherstellung zur Verringerung der Narbenbildung oder zur Steuerung der Folge von Vorgängen, die für die natürliche Gewebewiederherstellung essentiell sind; einen Wachstumsfaktor; einen Fibroblast-Wachstumsfaktor, einen Epithel-Wachstumsfaktor, oder einen transformierenden Wachstumsfaktor.

5. Verfahren nach einem der Ansprüche 1 bis 3, wobei die Flüssigkeit ein Elektretpolymer enthält und die Beaufschlagung mit dem elektrischen Feld einen Elektretpolymer-Faserstoff erzeugt, der von dem Substrat angezogen wird und sich auf diesem ablagert, um Zellkerne zu bilden oder interaktive Zell- und/oder Molekular-Vorgänge bei der Gewebewiederherstellung einzuleiten.

6. Verfahren nach einem der vorhergehenden Ansprüche, wobei die Beaufschlagung mit dem elektrischen Feld unter Verwendung eines Handgerätes (1a) erfolgt, das ein Gehäuse (9) mit einer mit einem Auslass (4) fluchtenden und in Abstand von dem Substrat (7) anzuordnenden Öffnung aufweist, wobei das Gehäuse (9) so ausgelegt ist, dass es sich von der Hand des Benutzers halten lässt und einen Vorratsbehälter (2a) mit der Flüssigkeit, einen Flüssigkeitszuführschlauch (3a) zur Zuführung von Flüssigkeit aus dem Vorratsbehälter an den Flüssigkeitsauslass (4), sowie eine Spannungsquelle (6) aufweist, die von dem Benutzer aktiviert wird, um an den Flüssigkeitsauslass (4) eine Spannung zur Erzeugung des elektrischen Feldes anzulegen.

7. Verfahren nach Anspruch 6, wobei die Gehäuseöffnung im Abstand von 5 bis 10 cm angeordnet wird.

8. Verfahren nach einem der vorhergehenden Ansprüche, wobei die Beaufschlagung mit dem elektrischen Feld bewirkt, dass die an dem Flüssigkeitsauslass (4) austretende Flüssigkeit einen Faserstoff mit einem Durchmesser im Bereich von 10 nm bis 10 µm bildet.

9. Verfahren nach einem der Ansprüche 1 bis 7, wobei die Zufuhr von Flüssigkeit aus dem Flüssigkeitsauslass (4) und/oder die Beaufschlagung der an dem Flüssigkeitsauslass (4) austretenden Flüssigkeit mit dem elektrischen Feld so gesteuert wird, dass die Flüssigkeit einen elektrisch geladenen Faserstoff mit einem Durchmesser im Bereich von 10 nm bis 10 µm und einem derartigen Ladungs/Masse-Verhältnis bildet, dass der Faserstoff von dem Substrat angezogen wird und sich unter Bildung eines gesteuerten Faserstoffmusters auf diesem anlagert.

10. Verfahren nach einem der Ansprüche 1 bis 8, wobei das Ladungs/MasseVerhältnis des Faserstoffs gesteuert wird, um zu ermöglichen, dass die Stelle, an der der Faserstoff auf das Substrat aufgebracht wird, hauptsächlich dadurch gesteuert wird, dass der Flüssigkeitsauslass (4) relativ zu dem Substrat bewegt und die Anzahl der Durchläufe und das Muster der Bewegung des Auslasses über das Substrat hinweg gesteuert werden.

11. Verfahren nach einem der vorhergehenden Ansprüche, wobei die Matte oder Bahn benachbarte Faserstoffteile aufweist, die durch einen Zwischenraum voneinander getrennt sind.

12. Verfahren nach einem der vorhergehenden Ansprüche, wobei durch Beaufschlagung mit dem elektrischen Feld der Flüssigkeitsstrom zum Flüssigkeitsauslass (4) so geregelt wird, dass die Flüssigkeit den Durchmesser des Faserstoffs steuert.

13. Verfahren nach einem der vorhergehenden Ansprüche, wobei der Faserstoff mindestens eine Faser und/oder Faserteile oder Fäserchen und/oder Teilchen enthält.

14. Verfahren nach einem der Ansprüche 1 bis 12, wobei durch Beaufschlagen mit dem elektrischen Feld bewirkt wird, dass die Matte oder Bahn elektrisch geladene Faserstoffteile enthält, oder anstelle der Matte oder Bahn elektrisch geladene Faserstoffteile vorgesehen werden, die bei Verwendung an einen Haut- oder weichen Gewebebereich elektrisch angezogen werden und an diesem haften, um die Substanz unter die Haut oder in das weiche Gewebe einzubringen.

15. Verfahren nach einem der vorhergehenden Ansprüche, wobei die Polarität des elektrischen Feldes geändert wird, um die Polarität, auf die der Faserstoff aufgeladen wird, zu ändern, so dass nacheinander Faserstofflagen entgegengesetzter Polarität auf das Substrat aufgetragen werden.

16. Verfahren nach einem der vorhergehenden Ansprüche, wobei zum Beaufschlagen mit dem elektrischen Feld Flüssigkeit, die an dem Flüssigkeitsauslass (4) austritt, dem elektrischen Feld unterworfen wird, während der Flüssigkeitsstrom zu dem Flüssigkeitsauslass (4) so geregelt wird, dass die von dem Flüssigkeitsauslass (4) austretende Flüssigkeit mehrere Flüssigkeitsstrahlen bildet, deren jeder Faserstoff erzeugt, der von dem Substrat elektrisch angezogen wird und sich auf diesem ablagert.

17. Verfahren nach einem der vorhergehenden Ansprüche, wobei das Substrat eine Vertiefung oder konkave Fläche aufweist und der elektrisch geladene Faserstoff vor Auftrag auf die Vertiefung oder konkave Fläche mindestens teilweise elektrisch entladen wird.

18. Verfahren nach einem der vorhergehenden Ansprüche, wobei die Flüssigkeit mindestens ein Polymer oder ein Lösungsmittel und ein Polymer enthält.

19. Verfahren nach einem der Ansprüche 1 bis 17, wobei dem Flüssigkeitsauslass (4) eine ein Polymer enthaltende Flüssigkeit zugeführt wird, wobei die Beaufschlagung mit dem elektrischen Feld einen elektrisch geladenen Polymerfaserstoff bildet, und wobei der elektrisch geladene Polymerfaserstoff in einem entgegengesetzt geladenen Sprühnebel aufgesprüht wird.

20. Verfahren nach Anspruch 18, wobei der Polymerfaserstoff in einem entgegengesetzt geladenen Sprühnebel aufgesprüht wird, der einen oberflächenaktiven Stoff enthält.

21. Verfahren nach einem der vorhergehenden Ansprüche, wobei die Beaufschlagung mit dem elektrischen Feld mindestens einen elektrisch geladenen Strahl erzeugt, der sich unter Bildung eines elektrisch geladenen gelartigen Faserstoffs teilweise verfestigt.

22. Verfahren nach einem der vorhergehenden Ansprüche, wobei durch Anlegen einer gepulsten Spannung bewirkt wird, dass der Strahl unter Bildung von Faserteilen aufbricht.

23. Verfahren nach einem der vorhergehenden Ansprüche, wobei das Substrat entweder ein Wundverbandsubstrat oder die Wunde selbst ist.

## Revendications

1. Un procédé de fourniture d'un pansement pour des plaies, le procédé comprenant les étapes consistant :
à fournir du liquide à une sortie de liquide (4) à proximité d'un substrat sur lequel il faut fournir le pansement pour les plaies ;
à soumettre le liquide sortant de la sortie de liquide (4) à un champ électrique pour obliger le liquide à former au moins un jet de liquide chargé électriquement, le liquide étant tel que, après sa formation, le au moins un jet forme un matériau fibreux chargé électriquement qui est attiré vers le substrat et s'y dépose pour former un mat où feuille sur le substrat ; et
munir le mat ou la feuille avec un matériau pour promouvoir la réparation tissulaire.

2. Un procédé selon la revendication 1, qui comprend la répétition de l'étape de soumission à un champ électrique afin de réaliser le mat ou la feuille sous forme d'un nombre de couches de matières fibreuses.

3. Un procédé selon la revendication 1 ou 2, qui comprend la fourniture du matériau par au moins un parmi : l'incorporation du matériau dans le mat ou la feuille ; l'incorporation du matériau dans au moins une couche de matière fibreuse où la feuille ou la feuille présente un nombre de couches de matière fibreuse ; l'incorporation du matériau dans la matière fibreuse ; la fourniture du matériau sur le mat ou la feuille ; la fourniture du matériau sur au moins une couche de matière fibreuse où le mat ou la feuille présente un nombre de couches de matière fibreuse ; la fourniture ou l'interversion du matériau entre les couches de matière fibreuse où le mat ou la feuille présente un nombre de couches de matière fibreuse ; la fourniture du matériau sur la matière fibreuse pendant le dépôt ; l'orientation d'un jet qui contient le matériau sur la feuille ou le mat et/ou sur au moins une couche de matière fibreuse où le mat ou la feuille présente un nombre de couches de matière fibreuse et/ou sur la matière fibreuse pendant le dépôt ; la fourniture du matériau dans le liquide alimenté à la sortie de liquide ; la pulvérisation électrohydrodynamique d'un liquide qui comprend le matériau.

4. Un procédé selon l'une quelconque des revendications précédentes, dans lequel le matériau comprend au moins un parmi : des cellules pour promouvoir la croissance de la peau ; des cellules de la peau ; des cytokines pour promouvoir la réparation tissulaire ou pour stimuler l'activité cytocinétique pour promouvoir les activités cellulaires afin de stimuler la croissance dendritique ; du matériau pour promouvoir la réparation tissulaire afin de diminuer la formation de cicatrices ou pour réguler autrement la séquence d'événements fondamentale à la réparation tissulaire naturelle ; un facteur de croissance ; un facteur de croissance de fibroblast, un facteur de croissance épithéliale, un facteur de croissance de transformation.

5. Un procédé selon l'une quelconque des revendications 1 à 3, dans lequel le liquide comprend un polymère de type électret ; et l'étape de soumission à un champ électrique forme de la matière fibreuse en polymère de type électret qui est attirée vers le substrat et s'y dépose pour fournir des nucléés ou d'initier autrement des événements cellulaires et/ou moléculaires interactifs dans le cadre de la réparation tissulaire.

6. Un procédé selon l'une quelconque des revendications précédentes, qui comprend la réalisation de l'étape de soumission à un champ électrique en utilisant un dispositif à mat (1a) comprenant un boîtier (9) présentant une ouverture alignée avec la sortie (4) et agencée pour être positionnée à distance du substrat (7), le boîtier (9) étant adapté pour être porté dans la main de l'utilisateur et le boîtier contenant :
un réservoir (2a) qui contient du liquide ; un tube de fourniture de liquide (3a) pour fournir un liquide à partir du réservoir vers la sortie de liquide (4) ; et une source de tension (6) qui est activée par l'utilisateur afin de provoquer l'application d'une tension afin de générer le champ électrique au niveau de la sortie de liquide (4).

7. Un procédé selon la revendication 6, dans lequel l'ouverture de boîtier est positionnée espacée d'une distance de 5 à 10 cm du substrat.

8. Un procédé selon l'une quelconque des revendications précédentes, dans lequel l'étape de soumission à un champ électrique provoque la formation de matière fibreuse par le liquide sortant de la sortie de liquide (4), la matière fibreuse présentant un diamètre compris dans la plage de 10 nanomètres à 10 micromètres.

9. Un procédé selon l'une quelconque des revendications 1 à 7, comprenant en outre la régulation d'au moins un parmi : la fourniture de liquide à partir de la sortie de liquide (4) et la soumission du liquide sortant de la sortie de liquide (4) au champ électrique pour provoquer la formation d'une matière fibreuse chargée électriquement à partir du liquide, la matière fibreuse présentant un diamètre dans la plage d'environ 10 nanomètres à 10 micromètres et présentant un rapport charge-à-masse tel que la matière fibreuse soit attirée au substrat et s'y dépose afin de former un motif régulé de matière fibreuse.

10. Un procédé selon l'une quelconque des revendications 1 à 8, qui comprend la régulation du rapport charge-à-masse de la matière fibreuse permettant ainsi de réguler l'emplacement où se dépose la matière fibreuse sur le substrat principalement par le déplacement de la sortie de liquide (4) par rapport au substrat et en régulant le nombre de passes ainsi que le modèle de déplacement de la sortie par-dessus le substrat.

11. Un procédé selon l'une quelconque des revendications précédentes, dans lequel le mat ou la feuille présente des parties adjacents de matière fibreuse espacées l'une de l'autre par un intervalle.

12. Un procédé selon l'une quelconque des revendications précédentes, dans lequel l'étape de soumission à un champ électrique comprend la régulation du débit de liquide vers la sortie de liquide (4) pour obliger le liquide à régler le diamètre de la matière fibreuse.

13. Un procédé selon l'une quelconque des revendications précédentes, dans lequel la matière fibreuse comprend au moins parmi : au moins une fibre ; des fragments de fibre ou des fibriles ; des particules.

14. Un procédé selon l'une quelconque des revendications 1 à 12, dans lequel la soumission au champ électrique provoque l'inclusion au sein du mat ou de la feuille de fragments de matière fibreuse chargée électriquement ou le remplacement du mat ou de la feuille par ces derniers, les fragments étant, lors de l'utilisation, attirés électriquement vers une zone de la peau ou du tissu mou pour y adhérer, afin de fournir du matériau en dessous de la peau ou au sein du tissu mou.

15. Un procédé selon l'une quelconque des revendications précédentes, comprenant en outre la variation de la polarité du champ électrique afin de changer la polarité à laquelle est chargée la matière fibreuse afin de provoquer le dépôt en succession de couches de polarité opposée de matière fibreuse sur le substrat.

16. Un procédé selon l'une quelconque des revendications précédentes, dans lequel l'étape de soumission soumet le liquide sortant de la sortie de liquide (4) à un champ électrique tout en régulant le débit de liquide vers la sortie de liquide (4) de sorte que le liquide qui sort de la sortie de liquide (4) forme une pluralité de jets de liquide dont chacun forme de la matière fibreuse qui est attirée électriquement au substrat pour s'y déposer.

17. Un procédé selon l'une quelconque des revendications précédentes, dans lequel le substrat comprend une cavité ou une surface concave et le procédé comprend en outre la décharge électrique partielle de la matière fibreuse chargée électriquement avant sa fourniture à la cavité ou sur la surface concave.

18. Un procédé selon l'une quelconque des revendications précédentes, dans lequel le liquide comprend un polymère ou un solvant et un polymère.

19. Un procédé selon l'une quelconque des revendications 1 à 17, dans lequel :
l'étape de fourniture comprend la fourniture de liquide qui contient un polymère à la sortie de liquide (4) ; l'étape de soumission forme de la matière fibreuse en polymère chargée électriquement ; et le procédé comprend en outre la pulvérisation de la matière fibreuse en polymère chargée électriquement avec un jet ou un nuage présentant une charge opposée.

20. Un procédé selon la revendication 18, qui comprend la pulvérisation de la matière fibreuse en polymère avec un jet présentant une charge opposée comprenant un agent tensioactif.

21. Un procédé selon l'une quelconque des revendications précédentes, dans lequel : l'étape de soumission génère au moins un jet chargé électriquement qui se solidifie partiellement afin de former une matière fibreuse de type gel chargée électriquement.

22. Un procédé selon l'une quelconque des revendications précédentes, comprenant en outre l'étape consistant à désintégrer le jet pour former des fragments de fibre par pulsation d'une tension appliquée.

23. Un procédé selon l'une quelconque des revendications précédentes, dans lequel le substrat est soit un substrat de pansement pour des plaies, soit la plaie elle-même.
